# EUROPÄISCHE PATENTSCHRIFT

(11) **EP 2 099 921 B1**
(45) Veröffentlichungstag und Bekanntmachung des Hinweises auf die Patenterteilung: **09.03.2011**
(21) Anmeldenummer: 07856085.1
(22) Anmeldetag: 11.12.2007
(51) Int. Cl.: C12P 3/00, C12P 5/02

(54) **Verfahren zur biologischen Erzeugung von Methan**
Process for the biological generation of methane
Procédé de production biologique de méthane

(30) Priorität: 11.12.2006 DE 102006058599; 08.01.2007 DE 102007002009; 20.06.2007 DE 102007028879; 06.07.2007 DE 102007031688
(43) Veröffentlichungstag der Anmeldung: 16.09.2009
(73) Patentinhaber: Salvetzki, Ralf, 30974 Wennigsen (DE)
(72) Erfinder: Salvetzki, Ralf, 30974 Wennigsen (DE)
(74) Vertreter: Ullrich & Naumann
(86) Internationale Anmeldenummer: PCT/DE2007/002234
(87) Internationale Veröffentlichungsnummer: WO 2008/071175

(56) Entgegenhaltungen:
- EP-A- 1 574 581
- WO-A-03/013748
- AU-A1- 2004 229 070
- US-A- 4 609 383
- US-B1- 6 391 256
- ROSENKRANS A M ET AL: "STIMULATION OF HYDROGEN PHOTOPRODUCTION IN ALGAE BY REMOVAL OF OXYGEN BY REAGENTS THAT COMBINE REVERSIBLY WITH OXYGEN" BIOTECHNOLOGY AND BIOENGINEERING, Bd. 26, Nr. 11, 1984, Seiten 1334-1342, XP002478930 ISSN: 0006-3592

## Beschreibung

Die vorliegende Erfindung betrifft ein Verfahren zur biologischen Erzeugung von Methan.

Methan ist ein wichtiger Energieträger sowie ein bedeutender Ausgangsstoff für die chemische Industrie. Methan wird ganz überwiegend aus Erdgas gewonnen, dessen Hauptbestandteil es ist. Dazu werden fossile Vorkommen ausgebeutet, in denen zumeist Erdgas und Erdöl zusammen vorliegen. Solche Vorkommen finden sich bspw. in Russland oder auch unter dem Meeresboden, bspw. in der Nordsee.

Die Verfügbarkeit solcher fossiler Erdgasvorkommen ist jedoch begrenzt. Des Weiteren kann eine wirtschaftliche Abhängigkeit von Erdgaslieferländem eintreten. Zusätzlich liegt das Methan im Erdgas neben einer Vielzahl anderer Komponenten vor und muss durch aufwändige Verfahren von den Beikomponenten abgetrennt werden, um reines Methan zu erhalten.

Das Hauptproblem bei der Nutzung von Methan aus fossilen Vorkommen ist jedoch zum einen das starke Treibhauspotential, welches Methan aufweist. Bei der Förderung und dem Transport von fossil vorkommendem Methan tritt unweigerlich ein erheblicher Verlust des abgebauten Gases ein, dieser Anteil gelangt als Treibhausgas in die Atmosphäre.

Zum anderen verstärkt die Nutzung (d.h. die Verbrennung) fossilen Methans den Treibhauseffekt dadurch, dass weiteres Kohlendioxid in die Atmosphäre gelangt und entsprechend als Treibhausgas wirken kann.

Es ist des Weiteren bekannt, methanhaltige Gase aus Biomasse zu gewinnen (sog. Biogas-Verfahren). So ist aus der DE 10 2004 035 997 A1 eine Biogasanlage zur Bereitstellung von methanhaltigen Gasen bekannt. Die Biogaserzeugung hat jedoch den Nachteil, dass nur sehr unreines Methan gewonnen wird, das durch Kohlendioxid, Wasserdampf, Ammoniak, Schwefelwasserstoff und andere Bestandteile verunreinigt ist. Des Weiteren ist nicht genügend Biomasse verfügbar, um letztlich die fossilen Vorkommen ersetzen zu können.

Schließlich ist bekannt, dass sich auf den Meeresböden der Erde mehrere Billionen Tonnen Methan in Form von Methanhydrat befinden. Die Förderung dieser Vorkommen ist bis jetzt jedoch noch nicht kommerziell möglich und scheint nur mit erheblichen Kosten realisierbar zu sein.

Der vorliegenden Erfindung liegt daher die Aufgabe zugrunde, ein umweltfreundliches und kostengünstiges Verfahren zur Erzeugung von Methan anzugeben, das auf die Nutzung fossiler Vorkommen verzichtet.

Erfindungsgemäß wird die voranstehende Aufgabe mit den Merkmalen des Patentanspruchs 1 gelöst. Danach weist das erfindungsgemäße Verfahren zur biologischen Erzeugung von Methan die folgenden Schritte auf:
- Erzeugung von Wasserstoff (H₂₎ und Sauerstoff (O₂) aus Kohlendioxid (CO₂) und Wasser (H₂O) durch Grünalgen unter Lichteinwirkung (Hydrogenese),
- Abtrennung der gasförmigen Zwischenprodukte Wasserstoff (H₂) und Sauerstoff (O₂) von dem Algenmedium über eine Membran (3),
- Abtrennung des erzeugten Sauerstoffs (O₂) vom erzeugten Wasserstoff (H₂),
- Erzeugung von Methan (CH₄) aus dem erzeugten Wasserstoff (H₂) und Kohlendioxid (CO₂) durch Methanogenese-Bakterien (Methanogenese),
- Abtrennung und ggf. Verflüssigung des erzeugten Methans (CH₄).

Vorteilhafte Ausgestaltungen des erfindungsgemäßen Verfahrens sind den nachgeordneten Patentansprüchen entnehmbar.

Erfindungsgemäß ist erkannt worden, dass die Darstellung von Methan in zwei Schritte aufteilbar ist, wobei in beiden Schritten jeweils eine umweltneutrale Bioreaktion stattfinden kann.

So wird zunächst durch den Einsatz von Grünalgen aus Kohlendioxid und Wasser Wasserstoff und Sauerstoff gewonnen.

Das eingesetzte Kohlendioxid kann aus der Umgebungsatmosphäre, bspw. durch ein Luftverflüssigungsverfahren mit anschließender Trockeneisbildung, gewonnen werden. Dadurch verringert sich der Anteil des treibhausaktiven Kohlendioxids in der Atmosphäre. Des Weiteren kann Kohlendioxid aus Industrie- oder Verbrennungsprozessen genutzt werden, wodurch die Kohlendioxidbelastung der Atmosphäre direkt verringert wird.

Der im ersten Schritt von den Grünalgen erzeugte Sauerstoff wird abgetrennt und kann anderen Verwendungen zugeführt werden. Es verbleibt der im ersten Schritt erzeugte Wasserstoff.

In erfindungsgemäßer Weise wird nun in einem zweiten Schritt aus dem erzeugten Wasserstoff und weiterem Kohlendioxid durch den Einsatz geeigneter Methanogenese-Bakterien Methan erzeugt. Dabei wird weiteres Kohlendioxid verbraucht.

Schließlich kann das erzeugte Methan von den verbliebenen Edukten Wasserstoff und Kohlendioxid abgetrennt und ggf. verflüssigt werden.

Durch den Verbrauch von Kohlendioxid während der erfindungsgemäßen Methangewinnung ist insgesamt ein kohlendioxidneutrales Verfahren angegeben. Dies bedeutet, dass das erzeugte Methan bei einer thermischen Umsetzung kein zusätzliches treibhausschädigendes Kohlendioxid produziert, da die entsprechende Kohlendioxidmenge bereits bei der Herstellung aus der Atmosphäre entnommen worden ist. Als Energieträger kann dabei Sonnenlicht verwendet werden, was die Energiebilanz des Verfahrens weiter verbessert. Das vorgeschlagene Verfahren kann daher eine Alternative zu den umweltfreundlichen Energiequellen Windenergie und Solarenergie werden.

Folglich ist ein umweltfreundliches und kostengünstiges Verfahren zur Gewinnung von Methan angegeben, das auf die Nutzung fossiler Vorkommen verzichtet.

In einer ersten Ausführungsform der Erfindung werden die zur Erzeugung von Wasserstoff verwendeten Algen in einer wässrigen Lösung bereitgestellt. Dieser Lösung werden ggf. periodisch oder fortlaufend geeignete Nährstoffe zugeführt. Dadurch wird den verwendeten Algen eine optimale Umgebung bereitgestellt und es ist ermöglicht, das erfindungsgemäße Verfahren ohne unerwünschte Unterbrechungen zu betreiben.

Die zur Erzeugung von Wasserstoff verwendeten Grünalgen können insbesondere Chlamydomonas reinhardtii aufweisen. Diese Algen eignen sich besonders zur Wasserstoffherstellung und harmonieren optimal mit den anderen Schritten des erfindungsgemäßen Verfahrens.

In einer Weiterbildung des Verfahrens werden die zur Erzeugung von Wasserstoff verwendeten Algen von der Lichtquelle, insbesondere dem Sonnenlicht, durch eine im Wesentlichen transparente Scheibe getrennt. Dadurch können die verwendeten Algen Sonnenlicht als Energieträger verwerten, ohne der Umgebung direkt ausgesetzt zu werden.

In bevorzugter Weise wird dabei eine Scheibe verwendet, die auf der lichtabgewandten Seite einen Selbstreinigungseffekt (Lotuseffekt) aufweist. Dieser Effekt kann bspw. durch eine Silanbeschichtung bereitgestellt werden. Die Verwendung einer solchen Scheibe stellt sicher, dass auf der Innenseite der Scheibe keine Algen aufwachsen oder sich Verschmutzungen festsetzen, welche ein Eindringen des Lichts auf die Innenseite der Scheibe verhindern könnten.

Des Weiteren ist erfindungsgemäß erkannt worden, dass Sauerstoff den Hydrogenese-Schritt intrazellulär stört. Daher wird ein Verfahren vorgeschlagen, bei dem - vorzugsweise bereits während des Hydrogenese-Schritts - der intrazelluläre Sauerstoff zumindest teilweise gebunden wird. Dadurch wird verhindert, dass sich Sauerstoff in den Zellen der verwendeten Algen anreichert und so die Wasserstoffproduktion hemmt.

Darüber hinaus ist vom vorliegenden Erfinder erkannt worden, dass neben einem zu hohen intrazellulären Sauerstoffgehalt auch eine zu hohe Lichteinstrahlung (über ca. 2000 W/m²) zur Bildung von Sauerstoffradikalen führt und dadurch ebenfalls die Hydrogenese hemmt. Zum Ausgleich dieses Effekts kann mit dem vorgeschlagenen Verfahren der Gehalt an intrazellulärem Sauerstoff gesenkt werden, um diesen unerwünschten Effekt auszugleichen. Dabei soll betont werden, dass - auch im Folgenden - der Begriff "intrazellulärer Sauerstoff" auch intrazellulär vorhandene Sauerstoffradikale umfasst, welche ebenfalls die Hydrogenese hemmen.

Der intrazelluläre Sauerstoff kann während oder nach dem Hydrogenese-Schritt gebunden werden. Mit anderen Worten kann der intrazelluläre Sauerstoff kontinuierlich bereits während der Entstehung abgefangen werden. Dies hat Vorteile in Bezug auf gleich bleibende Reaktionsbedingungen. Andererseits kann der intrazelluläre Sauerstoff von Zeit zu Zeit oder auch erst während der Dunkelphase der Hydrogenese (insbesondere bei Nacht) gebunden werden. Bei dieser Vorgehensweise tritt keine Störung bzw. Unterbrechung der Wasserstoffproduktion ein.

Darüber hinaus ist erkannt worden, dass auch in den Methanogenese-Bakterien intrazellulär vorhandener Sauerstoff die Methanogenese behindert. Deswegen wird im Folgenden auch eine bevorzugte Ausführungsform des Verfahrens vorgeschlagen, bei der der intrazelluläre Sauerstoff der Methanogenese-Bakterien ebenfalls gebunden wird. Die nachfolgend anhand der Algen vorgeschlagenen vorteilhaften Ausführungsformen zur Bindung des intrazellulären Sauerstoffs sind deswegen ausdrücklich nicht auf den Hydrogenese-Schritt beschränkt. Vielmehr eignen sich diese Weiterbildungen auch in vorteilhafter Weise zur Bindung des intrazellulären Sauerstoffs der Methanogenese-Bakterien und werden auch diesbezüglich als Ausgestaltungen des erfindungsgemäßen Verfahrens vorgeschlagen.

Es wird eine Ausführungsform bevorzugt, bei der der intrazelluläre Sauerstoff durch Zugabe zumindest eines Bindemittels gebunden wird.

Dabei wird bevorzugt, dass das bzw. die Bindemittel nach der Aufnahme von Sauerstoff regeneriert wird bzw. werden. Dadurch kann im günstigsten Fall das Bindemittel in der Zelle verbleiben und dort erneut wirksam werden. Des Weiteren wird damit ein kostengünstiges und umweltfreundliches Verfahren realisiert.

Der intrazelluläre Sauerstoff kann biochemisch gebunden werden.

In diesem Zusammenhang wird zunächst ein Verfahren vorgeschlagen, bei dem als Bindemittel Myoglobin zugegeben wird. Hierbei kann biochemisch hergestelltes Myoglobin in den Hydrogenese-Bioreaktor eingebracht werden. Das Eindringen des Myoglobins in die Algenzellen kann dadurch begünstigt werden, dass die Membranen der Algen mittels Elektroporation geöffnet werden. Bei der Elektroporation werden die Zellen kurz starken elektrischen Feldern ausgesetzt, wodurch die Plasmamembranen vorübergehend permeabel werden. Dadurch kann Myoglobin besonders einfach in die Zellen eindringen und den intrazellulären Sauerstoff binden. In einer weiteren Ausgestaltung wird das Myoglobin mittels DNA-Transkriptionstechnik gentechnisch in das Genom der Algen eingebracht, so dass das Myoglobin künftig von der Zelle selber hergestellt werden kann.

In einer Weiterbildung des Verfahrens wird als Bindemittel Porphorin zugegeben. Dabei werden bevorzugt Porphorin-Eisenkomplexe in den Hydrogenese-Bioreaktor eingebracht. Dabei können die Porphorine sterisch gehindert sein, so dass die Sauerstoffaufnahme eine Reduktion des Eisens verhindert. Auch dabei kann das Eindringen des Bindemittels in die Algenzelle durch Elektroporation begünstigt werden.

In Bezug auf die vorgenannten Weiterbildungen des Verfahrens wird eine Ausgestaltung bevorzugt, bei der das Myoglobin und/oder das Porphorin nach der Aufnahme von Sauerstoff elektrochemisch und/oder biochemisch und/oder physikalisch regeneriert wird bzw. werden. Zunächst kann das Myoglobin bzw. können die Porphorin-Eisenkomplexe mittels elektrochemischer Reduktion zu Eisenion (Fe²⁺)-Komplexen reduziert werden. Diese elektrochemische Reduktion kann bevorzugt während der Dunkelphase (bei Nacht) durchgeführt werden. Des Weiteren lassen sich Myoglobin und Porphorin-Eisenkomplexe mittels biochemischer Reduktion mit dem Enzym NADPH zu den bereits genannten Eisenion-Komplexen reduzieren. Schließlich kann Myoglobin bevorzugt während der Dunkelphase (bei Nacht) physikalisch regeneriert werden, in dem es mit Kohlendioxid beaufschlagt wird. Die Kohlendioxid-Moleküle verdrängen bei Vorliegen eines genügend hohen Partialdrucks die Sauerstoffmoleküle aus dem Myoglobin und nehmen deren Plätze ein.

In einer weiteren bevorzugten Ausführungsform des erfindungsgemäßen Verfahrens wird der intrazelluläre Sauerstoff chemisch gebunden.

Dabei wird zunächst bevorzugt, dass als Bindemittel Hydrazin und/oder ein Hydrazinsalz, insbesondere Eisen-Hydrazin, zugegeben wird. Das Hydrazin wird direkt in den Hydrogenese-Bioreaktor eingebracht. Das Hydrazin oder dessen Salze entfalten eine reduzierende Wirkung, so dass intrazellulärer Sauerstoff oder Sauerstoffradikale gebunden werden.

Eine weitere Möglichkeit der chemischen Bindung von intrazellulärem Sauerstoff besteht drin, dass als Bindemittel ein Terpen, insbesondere α-Terpen und/oder Isopren, und/oder ein Derivat hiervon zugegeben wird. Terpene weisen im Allgemeinen eine reduzierende Wirkung auf, so dass intrazellulärer Sauerstoff oder Sauerstoffradikale gebunden werden. In besonders bevorzugter Weise wird zu dem Terpen noch der Triphenylmethan-Farbstoff Eosin und/oder ein Hämprotein der Cytochrom P450-Familie zugegeben. Diese Substanzen verstärken als sogenannte Photosensitizer die reduzierende Wirkung des Terpens.

In einer bevorzugten Ausführungsform werden die gasförmigen Zwischenprodukte Wasserstoff und Sauerstoff von dem Algenmedium durch eine poröse Membran aus CLPE (cross-linked polyethylene) abgetrennt. Dabei können die gasförmigen Zwischenprodukte durch die Membran diffundieren, während das in der Regel wässrige Algenmedium durch die Membran am Durchtritt gehindert wird. Dabei ist festgestellt worden, dass sich die genannte Membran aus CLPE besonders gut zur Abtrennung der vorliegenden Mischungspartner eignet.

In einer Weiterbildung dieser Ausführungsform wird die Membran beidseitig in eine hexagonal dichteste Kugelpackung festgelegt. Dadurch wird eine besonders sichere Fixierung der Membran erreicht.

In einer weiteren Ausführungsform wird eine CLPE-Membran mit einer inneren Schicht aus PATBS (Poly(acrylamid-tert-butyl-suffonsäure)) verwendet. Die innere Schicht aus PATBS erhöht in weiter vorteilhafter Weise die Leistungsfähigkeit der vorgeschlagenen CLPE-Membran für die Abtrennung der vorliegenden Mischungspartner.

In diesem Zusammenhang wird eine Weiterbildung des Verfahrens bevorzugt, bei dem einen mehrlagige Membran verwendet wird, deren Lagen zumindest bereichsweise miteinander verscheißt sind, insbesondere in einem Kreis- oder Wabenmuster. Durch das Verschweißen der verschiedenen Lagen der Membran wird eine erhöhte Druckfestigkeit bereitgestellt, so dass sich der Gasdruck in vorteilhafter Weise steigern lässt.

In einer weiteren Ausgestaltung wird im Hydrogenese-Schritt eine schwarze Membran verwendet. Die schwarze Membran absorbiert einen Großteil des eingestrahlten Lichts, so dass eine Erwärmung der Membran und des umliegenden Algenmediums erreicht wird. Durch die Wärmeentwicklung wird die Diffusion sowohl des Sauerstoffs als auch des Wasserstoffs begünstigt. Zusätzlich kann die Wärmeenergie abgeführt und anderweitig genutzt werden, beispielsweise durch einen Wärmeübertrager. Des Weiteren ist es möglich, die schwarze Membran direkt an ein Wärmeübertragemittel anzuschließen, so dass die eingestrahlte Wärmeenergie direkt abführbar ist.

Da das Kohlendioxid von den verwendeten Algen umgesetzt wird, ist ein Durchtritt des Kohlendioxids auf die Gasseite der Membran (d.h. in Richtung der gasförmig vorliegenden Mischung aus Wasserstoff und Sauerstoff) unerwünscht. Daher kann der osmotische Druck von Kohlendioxid auf der Gasseite der Membran erhöht werden, damit im Algenmedium eine erwünschte Mindestkonzentration von Kohlendioxid aufrechterhalten wird.

Die im ersten Schritt gewonnenen gasförmigen Mischungspartner Sauerstoff und Wasserstoff werden in bevorzugter Weise durch eine Gasverflüssigung, insbesondere nach dem Linde-Verfahren, voneinander getrennt Das Linde-Verfahren ist für sich gesehen bekannt. Dabei wird ein Gas bzw. eine Gasmischung so lange abgekühlt, bis die einzelnen Mischungspartner ihren Siedepunkt erreichen und als Flüssigkeit anfallen. In diesem Fall liegt der Siedepunkt von Sauerstoff ca. 70 Kelvin oberhalb des Siedepunkts von Wasserstoff, so dass der Sauerstoff zuerst flüssig wird. Die Verwendung eines Gasverflüssigungsverfahrens ist besonders vorteilhaft, wenn sehr reine Komponenten gewonnen werden sollen.

Aus reinem Sauerstoff und reinem Wasserstoff kann so als Nebenprodukt des Verfahrens reinstes Süßwasser erhalten werden, insbesondere unter Nutzung von Abwärme aus dem Hydrogenese-Schritt.

Es wird insgesamt eine Ausführungsform der Erfindung bevorzugt, bei der auch die zur Erzeugung von Methan verwendeten Methanogenese-Bakterien in einer wässrigen Lösung bereitgestellt werden. Dieser Lösung können des Weiteren periodisch oder fortlaufend geeignete Nährstoffe zugeführt werden. Damit wird für die verwendeten Methanogenese-Bakterien eine optimale Umgebung bereitgestellt. Des Weiteren ist eine ununterbrochene Durchführung des erfindungsgemäßen Verfahrens ermöglicht.

In einer weiteren Ausführungsform weisen die zur Erzeugung von Methan verwendeten Methanogenese-Bakterien eine oder eine Mischung der Arten Methanobacterium thermoautotropicum, Methanobacillus, Methanobacterium, Methanococcus, Methanosarcina und Methanothrix auf. Es ist festgestellt worden, dass die genannten Bakterienarten besonders gut mit den anderen Schritten des erfindungsgemäßen Verfahrens harmonieren.

Im zweiten Schritt des Verfahrens kann der Wasserstoff und das Kohlendioxid den Methanogenese-Bakterien unter anaeroben Bedingungen und/oder bei einer Temperatur von ungefähr 60°C zugeführt werden. Die genannten Bedingungen haben sich als optimal erwiesen, um eine möglichst hohe Methanausbeute in Verbindung mit einer langen Lebensdauer und einer besonders gleichmäßigen Aktivität der verwendeten Bakterien bereitzustellen.

Es ist des Weiteren erkannt worden, dass intrazellulärer Sauerstoff bzw. intrazellulär vorliegende Sauerstoffradikale nicht nur die Wasserstoffproduktion der Algen hemmen, sondern ebenfalls die Produktivität der Methanogenese-Bakterien herabsetzen. Daher besteht eine besonders bevorzugte Weiterbildung des erfindungsgemäßen Verfahrens darin, den Gehalt an intrazellulärem Sauerstoff innerhalb der Methanogenese-Bakterien ebenfalls herabzusetzen. Dazu werden ausdrücklich alle Maßnahmen vorgeschlagen, die als bevorzugte Ausführungsformen zu Reduktion des intrazellulären Sauerstoffs innerhalb der Algen des Hydrogenese-Schritts angesprochen worden sind. Mit anderen Worten eignen sich sämtliche dieser Maßnahmen auch in vorteilhafter Weise zur Anwendung auf die Methanogenese-Bakterien, finden jedoch zur Vermeidung von Wiederholungen keine erneute detaillierte Erwähnung.

Es wird eine Ausführungsform bevorzugt, bei der die Abtrennung des Methans von dem Bakterienmedium über eine Membran, insbesondere über eine poröse Membran aus CLPE (cross-linked polyethylene), erfolgt.

Auch die Membran des zweiten Schrittes kann in eine hexagonal dichteste Kugelpackung festgelegt werden.

Dabei wird die Verwendung einer Membran bevorzugt, die eine innere Schicht aus PATBS aufweist.

Auch hier kann in vorteilhafter Weise eine mehrlagige Membran verwendet werden, deren Lagen zumindest bereichsweise miteinander verschweißt sind, insbesondere in einem Kreis- oder Wabenmuster.

Bezüglich der letztgenannten Ausführungsformen wird zur Vermeidung von Wiederholungen auf die Ausführungen in Bezug auf vorteilhafte Merkmale der im ersten Schritt verwendeten Membran verwiesen.

Auch in Bezug auf den zweiten Reaktionsschritt wird eine Weiterbildung des erfindungsgemäßen Verfahrens vorgeschlagen, bei der der osmotische Druck des Kohlendioxids auf der Gasseite der Membran erhöht wird. Dadurch lässt sich eine gleichbleibend hohe Konzentration von Kohlendioxid im Bakterienmedium sicherstellen. Dies ist vorteilhaft, da das Kohlendioxid von den Bakterien letztlich zu Methan umgesetzt wird.

Es wird weiterhin eine Ausgestaltung des Verfahrens vorgeschlagen, bei der die Abtrennung des im zweiten Schritt erzeugten Methans von dem Wasserstoff durch eine Gasverflüssigung, insbesondere nach dem Linde-Verfahren, erfolgt. In Bezug auf die Vorteile dieser Ausführungsform kann auf die Erläuterungen bezüglich der Auftrennung von Sauerstoff und Wasserstoff verwiesen werden.

In Bezug auf beide Schritte des erfindungsgemäßen Verfahrens, bei denen jeweils Kohlendioxid verbraucht wird, wird vorgeschlagen, dass das den Algen und/oder den Methanogenese-Bakterien zugeführte Kohlendioxid aus Trockeneis gewonnen wird, wobei das Trockeneis ggf. aus einer Luftverflüssigung, insbesondere nach dem Linde-Verfahren, gewonnen wird. So lässt sich Kohlendioxid bzw. Trockeneis bereitstellen, das praktisch keine Verunreinigungen beinhaltet. Des Weiteren wird der Umgebungsluft Kohlendioxid entnommen, was eine vorteilhafte Auswirkung auf den Treibhauseffekt hat.

Alternativ oder zusätzlich kann das den Algen und/oder den Methanogenese-Bakterien zugeführte Kohlendioxid aus kohlendioxidreichen Gasströmen, insbesondere aus Industrie- und Verbrennungsprozessen, bereitgestellt werden. Diese Weiterbildung des Verfahrens ist insbesondere im Hinblick auf die Verbesserung der Kohlendioxidbilanz von Müllverbrennungs- und Kraftwerksanlagen vorteilhaft. So kann aus eigentlich umweltschädlichen Abgasströmen mit Hilfe des vorliegenden Verfahrens Methan bereitgestellt werden, das als Ausgangsstoff für die chemische Industrie oder zur kohlendioxidneutralen Verbrennung genutzt werden kann.

Im Hinblick auf eine Verbesserung der Energiebilanz und eine Verminderung des Ausstoßes von treibhausaktivem Kohlendioxid wird eine Weiterbildung des Verfahrens vorgeschlagen, bei dem nicht verbrauchtes bzw. nicht umgesetztes Kohlendioxid - insbesondere mittels einer Kühlfalle - zurückgewonnen und in den Prozess rückgeführt wird.

Schließlich ist im Hinblick auf eine zusätzlich verbesserte Methanausbeute des Verfahrens eine Ausgestaltung bevorzugt, bei der entstehendes überschüssiges Algen- und/oder Bakterienmaterial periodisch oder fortlaufend dem Prozess entnommen und einem Biogasprozess zur zusätzlichen Methangewinnung zugeführt wird. Bei der Durchführung des erfindungsgemäßen Verfahrens entsteht fortlaufend Biomasse, die aufgrund der festgelegten Anlagengröße von Zeit zu Zeit oder fortlaufend entfernt werden muss. Bei dieser Weiterbildung des Verfahrens kann diese Biomasse, die ansonsten - ggf. mit weiteren Folgekosten - entsorgt werden müsste, einer weiteren sinnvollen Verwendung zugeführt werden.

Im Sinne einer optimierten Energiebilanz des erfindungsgemäßen Verfahrens kann Wärme aus dem Hydrogenese-Schritt abgeführt werden, insbesondere durch einen Wärmeübertrager oder einer Wärmepumpe. Zum Erhalt einer besonders großen Wärmemenge kann - wie bereits erwähnt - eine schwarze Membran im Hydrogenese-Schritt verwendet werden. Die überschüssige Wärme lässt sich sinnvoll weiterverwenden oder innerhalb des Verfahrens übertragen.

Dabei kann Wärme zwischen dem Hydrogenese-Schritt und dem Methanogenese-Schritt übertragen werden. Mit anderen Worten lässt sich Abwärme aus dem Hydrogenese-Schritt in vorteilhafter Weise für die Methanogenese verwenden. Da die Methanogenese jedoch im Allgemeinen auf einem deutlich höheren Temperaturniveau stattfindet als die Hydrogenese, bietet sich hierbei die Verwendung einer Wärmepumpe an.

Es gibt nun verschiedene Möglichkeiten, die Lehre der vorliegenden Erfindung in vorteilhafter Weise auwzugestalten und weiterzubilden. Dazu ist einerseits auf die nachgeordneten Ansprüche, andererseits auf die nachfolgende Erläuterung eines bevorzugten Ausführungsbeispiels des erfindungsgemäßen Verfahrens anhand der Zeichnung zu verweisen. In Verbindung mit der Erläuterung des bevorzugten Ausführungsbeispiels anhand der Zeichnung werden auch im Allgemeinen bevorzugte Ausgestaltungen und Weiterbildungen der Lehre erläutert. In der Zeichnung zeigen
- Fig. 1: eine schematische Darstellung des Teilschrittes der Ausführungsform des erfindungsgemäßen Verfahrens, bei dem Wasserstoff und Sauer- stoff erzeugt wird,
- Fig. 2: eine schematische Darstellung des Teilschrittes der Ausführungsform des erfindungsgemäßen Verfahrens, bei dem Methan gewonnen wird,
- Fig. 3: eine schematische Darstellung des gesamten Verfahrens gemäß ge- zeigter Ausführungsform und
- Fig. 4: eine schematische Darstellung einer bevorzugten Membrananordnung zur Abtrennung gasförmiger (Zwischen-)Produkte von dem Algen- bzw. Bakterienmedium, und
- Fig. 5: eine schematische Darstellung zweier Ausführungsformen von mehr- lagigen Membranen, die bevorzugt zur Gasabtrennung im erfindungs- gemäßen Verfahren verwendet werden.

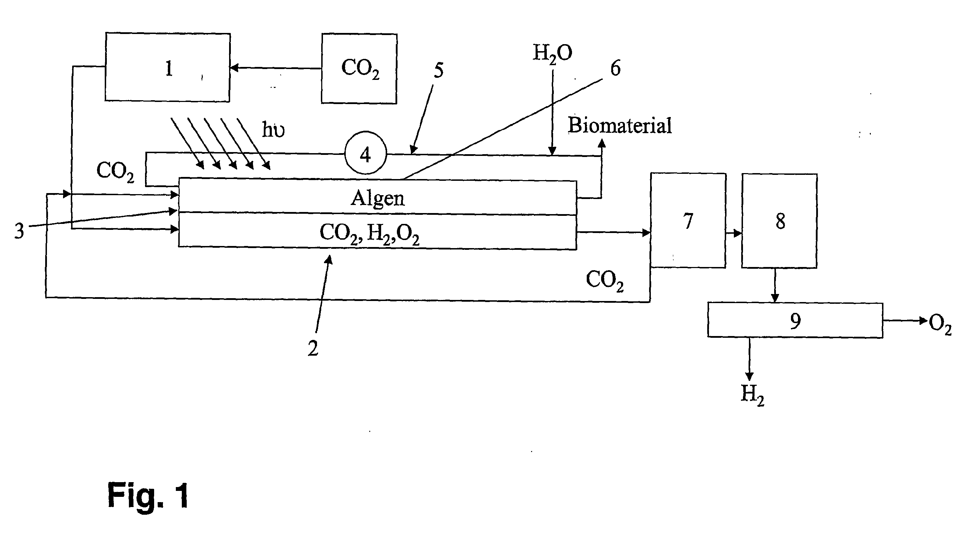

Fig. 1 zeigt eine schematische Darstellung eines Teils der vorgeschlagenen Ausführungsform des Gesamtverfahrens. Dieser erste Teil bezieht sich auf die Erzeugung von Wasserstoff und Sauerstoff durch Algen und die Abtrennung des erzeugten Sauerstoffs vom Wasserstoff.

Zu Beginn des Verfahrens wird Kohlendioxid (CO₂) bereitgestellt. Dieses Kohlendioxid kann bspw. aus einer Gasverflüssigung nach dem Linde-Verfahren bereitgestellt werden. Das Kohlendioxid kann aus der Gasverflüssigung direkt als Trockeneis entnommen werden. Alternativ wird gasförmiges Kohlendioxid (CO₂) in einer Trockeneiserzeugung 1 zu Trockeneis umgewandelt. -

Das Kohlendioxid (CO₂) wird in einen Bioreaktor 2 eingeleitet. In diesem Bioreaktor liegt eine wässrige Lösung aus Grünalgen (Chlamydomonas reinhardtii) vor. Die wässrige Grünalgenlösung ist vom Gasraum des Bioreaktors 2 durch eine Membran 3 aus CLPE getrennt. Das bereitgestellte Kohlendioxid wird in die wässrige Algenlösung eingeleitet und stets mit einer Pumpe 4 umgepumpt, also im Kreislauf geführt. Des Weiteren werden der wässrigen Lösung aus Grünalgen fortlaufend Nährstoffe 5 sowie Wasser (H₂O) zugeführt.

Die Algen werden von der Umgebung durch eine im Wesentlichen transparente Scheibe 6 getrennt. Aufgrund der Einstrahlung von Licht (hu), insbesondere dem Sonnenlicht, und durch die Versorgung der Algen mit Nährstoffen, Wasser und Kohlendioxid produzieren die verwendeten Grünlagen Wasserstoff (H₂) und Sauerstoff (O₂). Diese Zwischenprodukte können durch die Membran 3 in den Gasraum des Bioreaktors 2 übertreten und abgezogen werden.

Zur Erhöhung des osmotischen Drucks von Kohlendioxid (CO₂) auf der Gasseite der Membran 3 wird dem Gasraum des Bioreaktors 2 ebenfalls Kohlendioxid (CO₂) zugeführt.

In weiter vorteilhafter Weise können nun intrazellulärer Sauerstoff und Sauerstoffradikale in den Algen gebunden werden. Dazu kann dem Bioreaktor 2 ein Bindemittel zugegeben werden. Geeignete Bindemittel sind beispielsweise Myoglobin, Porphorin, Hydrazin oder Terpene. Solche Bindemittel können in die Algenzellen eindringen und dort den interzellulären Sauerstoff binden. Das Eindringen des Bindemittels in die Zelle kann mittels Elektroporation erleichtert werden. In diesen Fall sind innerhalb des Bioreaktors 2 Elektroden angebracht, die hier nicht dargestellt sind. Die verwendeten Bindemittel können auf verschiedene Weise regeneriert werden, wozu auf die vorangegangenen Ausführungen Bezug genommen wird. Sofern das oder die Bindemittel elektrochemisch regeneriert werden, sind im Bioreaktor 2 ebenfalls Elektroden angeordnet, welche hier nicht dargestellt sind. Dabei können für die Elektroporation und die elektrochemische Regeneration gegebenenfalls dieselben Elektroden verwendet werden.

Mit den Zwischenprodukten Wasserstoff (H₂) und Sauerstoff (O₂) aus dem Bioreaktor 2 austretendes Kohlendioxid (CO₂) wird in einer Kühlfalle 7 abgetrennt und in den Kreislauf rückgeführt.

Die Auftrennung der Zwischenprodukte Sauerstoff (O₂) und Wasserstoff (H₂) erfolgt über eine Gasverflüssigung 8 und über eine Fraktionierung 9.

Aufgrund des stetigen Wachstums der Algen im Bioreaktor 2 kann diesen fortlaufend oder periodisch überschüssige Biomaterial entnommen werden. Dieses Biomaterial wird in einem Biogasverfahren zur weiteren Methangewinnung verwendet.

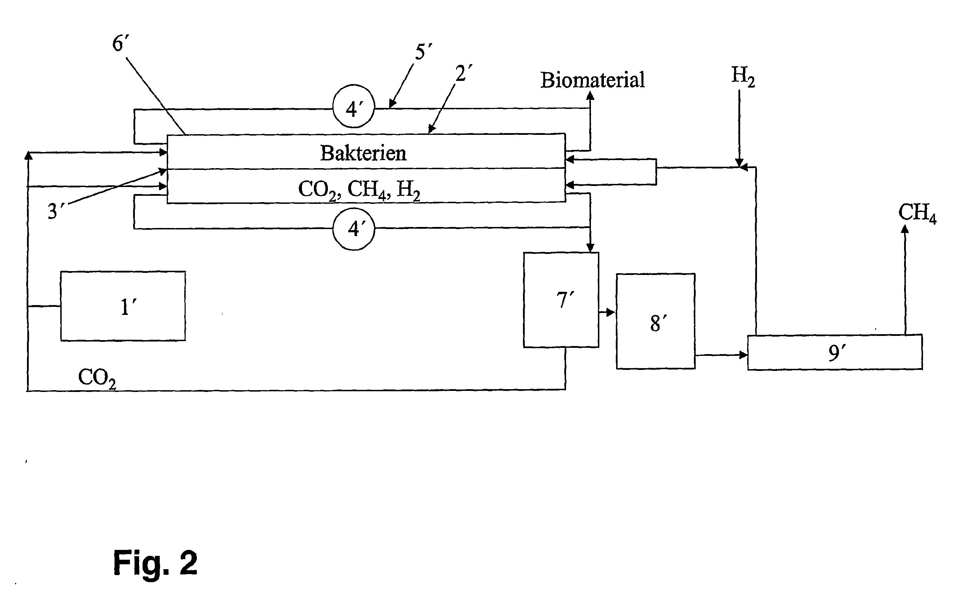

Dem Bioreaktor 2 kann fortlaufend überschüssige Wärme entnommen werden. Dazu wird bevorzugt ein Wärmeübertrager verwendet (nicht dargestellt). Des Weiteren kann im Hinblick auf eine verbesserte Energiebilanz Wärme aus dem Bioreaktor 2 mittels einer Wärmepumpe (nicht dargestellt) abgezweigt und dem Bioreaktor 2' für die Methanogenese (siehe Fig. 2) auf einem höheren Temperaturniveau zugeführt werden.

Fig. 2 zeigt eine schematische Darstellung des zweiten Schrittes der gezeigten Ausführungsform des erfindungsgemäßen Verfahrens, der sich auf die Erzeugung von Methan aus dem im ersten Schritt erzeugten Wasserstoff und die Abtrennung des gewonnenen Methans bezieht.

Der im ersten Schritt gewonnene Wasserstoff (H₂) wird einem weiteren Bioreaktor 2' zugeführt. In diesem Bioreaktor 2' liegt eine wässrige Lösung von Methanogenese-Bakterien vor, die von dem Gasraum des Bioreaktors 2' durch eine Membran 3' abgetrennt ist. Die Membran 3' besteht wie für den ersten Schritt gemäß Fig. 1 aus CLPE. Den in wässriger Lösung vorliegenden Methanogenese-Bakterien wird sowohl Wasserstoff (H₂) als auch Kohlendioxid (CO₂) zugeführt. Dazu wird - bspw. aus einem Gasverflüssigungsverfahren stammendes - Kohlendioxid in der Trockeneiserzeugung 1' in Trockeneis umgewandelt.

Die Edukte Kohlendioxid (CO₂) und Wasserstoff (H₂) werden der Bakterienlösung unter anaeroben Bedingungen bei einer Temperatur von ungefähr 60°C zugeführt. Des Weiteren werden der Bakterienlösung fortlaufend geeignete Nährstoffe 5' zugegeben. Sowohl die Bakterienlösung als auch die Gase im Gasraum des Bioreaktors 2' werden fortlaufend durch Pumpen 4' umgewälzt.

In erfindungsgemäßer Weise produzieren die Methanogenese-Bakterien aus dem zugeführten Wasserstoff (H₂) und Kohlendioxid (CO₂) durch die Einstellung geeigneter Umgebungsbedingungen und durch die Zuführung von Nährstoffen 5' Methan (CH₄). Das entstandene Methan kann durch die Membran 3' aus CLPE in den Gasraum des Bioreaktors 2' diffundieren. Zur Verhinderung einer übermäßigen Diffusion von Wasserstoff (H₂) und Kohlendioxid (CO₂) in den Gasraum wird der osmotische Druck der beiden Edukte auf der Gasseite der Membran 3' erhöht.

Obwohl die Methanogenese prinzipiell unter anaeroben Bedingungen durchgeführt wird, kann es in den Methanogenese-Bakterien intrazellulär zum Auftreten oder sogar zu einer Anreicherung von Sauerstoff und/oder von Sauerstoffradikalen kommen. Dieser intrazelluläre Sauerstoff behindert jedoch die Methanerzeugung. Deswegen wird als vorteilhafte Ausführungsform vorgeschlagen, den intrazellulären Sauerstoff der Methanogenese-Bakterien zu binden. Dazu kann - wie bei den Hydrogenese-Algen - ein geeignetes Bindemittel zugegeben werden. Solch ein Bindemittel kann beispielsweise biochemisch oder chemisch wirken, um den intrazellulären Sauerstoff zu binden. Geeignete Bindemittel umfassen Myoglobin, Porphorin, Hydrazin oder Terpene.

In bevorzugter Weise wird das Bindemittel nach der Aufnahme von intrazellulärem Sauerstoff regeneriert. Dabei kann wie bezüglich der Hydrogenese-Algen beschrieben vorgegangen werden.

Das Gasgemisch aus Kohlendioxid (CO₂), Wasserstoff (H₂) und Methan (CH₄) kann aus dem Gasraum des Bioreaktors 2' entnommen werden. Danach wird zunächst in einer Kühlfalle 7' das Kohlendioxid (CO₂) abgetrennt und in den Kreislauf rückgeführt. Die Auftrennung der verbleibenden Komponenten Wasserstoff (H₂) und Methan (CH₄) erfolgt über eine Gasverflüssigung 8' und eine nachgeschaltete Fraktionierung 9'. Der abgetrennte Wasserstoff (H₂) wird ebenfalls in den Prozess rückgeführt.

Übrig bleibt das Produkt Methan (CH₄) in hoher Reinheit. -

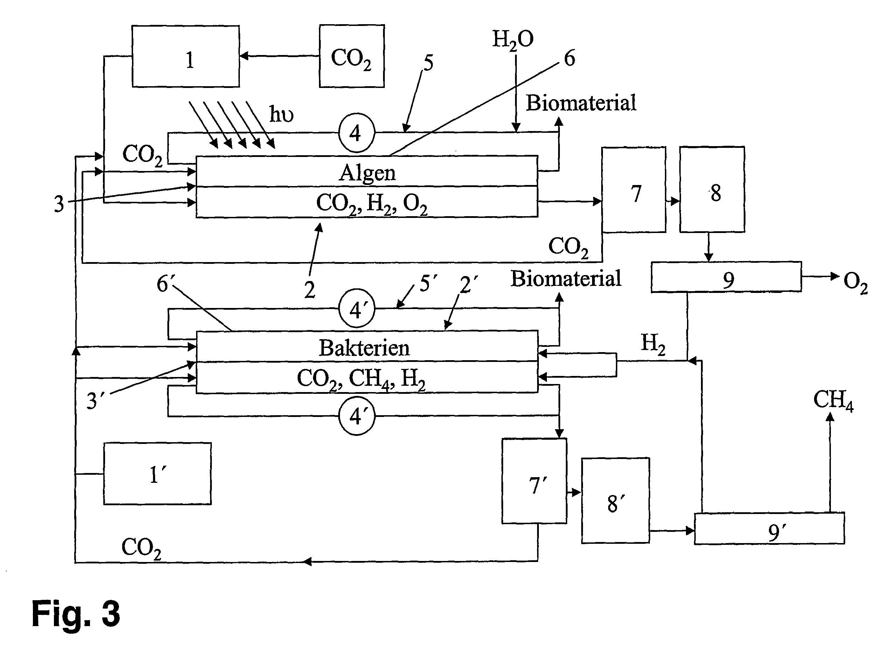

Fig. 3 zeigt eine schematische Darstellung des gesamten Verfahrens. Dabei wird deutlich, wie der im ersten Schritt gewonnene Wasserstoff (H₂) als Edukt für den zweiten Schritt übergeleitet und verwendet wird. Des Weiteren wird im zweiten Schritt zurückgewonnenes Kohlendioxid (CO₂) auch in den ersten Schritt rückgeführt.

Auch im zweiten Schritt entsteht durch das stetige Wachstum der verwendeten Methanogenese-Bakterien andauernd überschüssiges Biomaterial, welches zur weiteren Methangewinnung im Biogas-Verfahren ausgeschleust wird.

Zu den weiteren einzelnen Verfahrensschritten wird zur Vermeidung von Wiederholungen auf die Ausführungen zu Fig. 1 und Fig. 2 verwiesen.

Beim erfindungsgemäßen Verfahren laufen folgende Brutto-Reaktionen ab:
1. Schritt (Fig. 1): 2 H₂O → 2 H₂ + O₂
2. Schritt (Fig. 2): CO₂ + 4 H₂ → CH₄ + 2 H₂O
gesamt (Fig. 3): CO₂ + 2 H₂O → 2 O₂ + CH₄

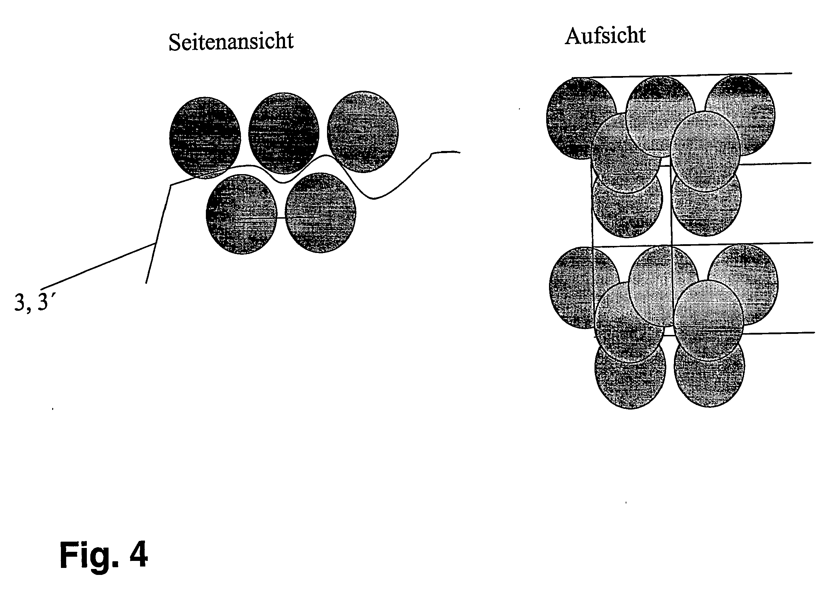

Fig. 4 zeigt eine schematische Darstellung einer bevorzugten Festlegung der erfindungsgemäß verwendeten Membrane 3, 3'. Dabei wird in dem Verfahren sowohl bei der Wasserstoffgewinnung durch Algen als auch bei der Methangewinnung durch Methanogenese-Bakterien vorzugsweise eine Membran 3, 3' aus CLPE verwendet, um den Übertritt von gasförmigen Produkten aus einem wässrigen Medium zu ermöglichen.

Diese Fig. zeigt in einer Seitenansicht und in einer Draufsicht, wie die Membran 3, 3' aus CLPE in einer hexagonal dichtesten Kugelpackung verankert wird, um eine möglichst sichere Festlegung der Membran 3, 3' zu erreichen.

Diese Art der Festlegung der Membran ist dabei im erfindungsgemäßen Verfahren bevorzugt, zur Ausführung des Verfahrens jedoch nicht zwingend notwendig.

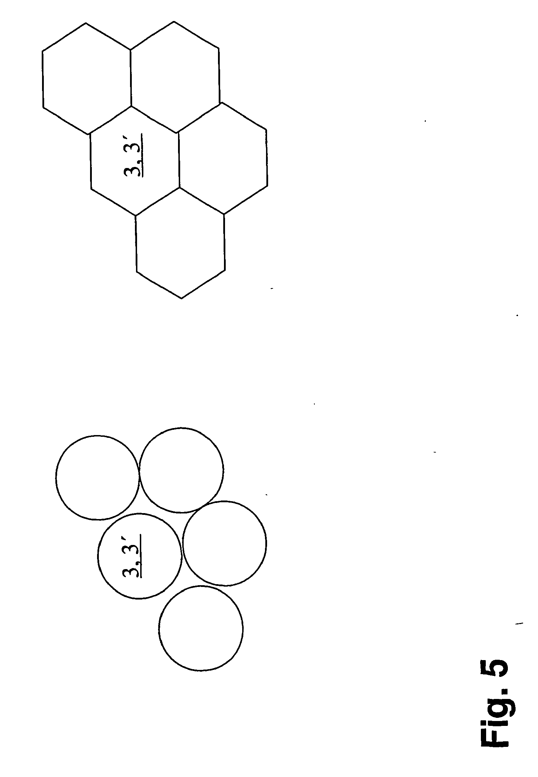

Fig. 5 zeigt eine schematische Darstellung zweier Ausführungsformen mehrlagiger Membranen, welche bevorzugt im erfindungsgemäßen Verfahren verwendet werden. Die Membranen 3, 3' bestehen aus zumindest zwei Lagen, können beispielsweise eine innere Schicht aus PATBS aufweisen. Zur Erhöhung der Druckfestigkeit sind die Lagen der Membranen 3, 3' zumindest bereichsweise miteinander verschweißt. In Fig. 5 links ist ein Kreismuster dargestellt. Die Kreise können sich berühren oder auch geringfügig voneinander beabstandet sein. In Fig. 5 rechts sind die verschiedenen Lagen der Membran 3, 3' in einem Wabenmuster miteinander verschweißt, wodurch eine erhöhte Druckstabilität erreicht ist.

Bei Verwendung der bevorzugten Membranen sowohl im Bioreaktor 2 als auch im Bioreaktor 2' kann der Druck auf der Gasseite der Membran erhöht werden, ohne dass diese versagt. Demzufolge ist insbesondere der osmotische Druck von Kohlendioxid weiter steigerbar.

Die Membran 3 im Bioreaktor 2 kann aus schwarzem Material gefertigt sein. Dadurch fällt im Bioreaktor 2 ein größerer Überschuss an Wärme an, die auf die bereits beschriebene Art und Weise vorteilhaft nutzbar ist.

Abschließend sei hervorgehoben, dass das voranstehend beschriebene Ausführungsbeispiel des erfindungsgemäßen Verfahrens die beanspruchte Lehre erörtert, diese jedoch nicht auf das Ausführungsbeispiel einschränkt.

### Bezugszeichenliste

- 1, 1': Trockeneiserzeugung
- 2, 2': Bioreaktor
- 3, 3': Membran
- 4, 4': Pumpe
- 5, 5': Nährstoffe
- 6, 6': Scheibe
- 7, 7': Kühlfalle
- 8, 8': Gas-/Luftverflüssigung
- 9, 9': Fraktionierung

## Patentansprüche

1. Verfahren zur biologischen Erzeugung von Methan (CH₄), wobei das Verfahren die Schritte aufweist:
- Erzeugung von Wasserstoff (H₂) und Sauerstoff (O₂) aus Kohlendioxid (CO₂) und Wasser (H₂O) durch ***Grünalgen*** unter Lichteinwirkung (Hydrogenese),
- ***Abtrennung der gasförmigen Zwischenprodukte Wasserstoff (H₂) und Sauerstoff (O₂) von dem Algenmedium über eine Membran (3),***
- Abtrennung des erzeugten Sauerstoffs (O₂) vom erzeugten Wasserstoff (H₂),
- Erzeugung von Methan (CH₄) aus dem erzeugten Wasserstoff (H₂) und Kohlendioxid (CO₂) durch Methanogenese-Bakterien (Methanogenese),
- Abtrennung und ggf. Verflüssigung des erzeugten Methans (CH₄).

2. Verfahren nach Anspruch 1, **dadurch gekennzeichnet, dass** die zur Erzeugung von Wasserstoff (H₂) verwendeten Algen von der Lichtquelle, insbesondere dem Sonnenlicht, durch eine im Wesentlichen transparente Scheibe (6) getrennt werden.

3. *Verfahren nach Anspruch 2, **dadurch gekennzeichnet, dass** eine Scheibe (6) verwendet wird, die auf der lichtabgewandten Seite einen Selbstreinigungseffekt (Lotuseffekt) aufweist, der durch eine Silanbeschichtung bereitgestellt wird.*

4. Verfahren nach einem der Ansprüche 1 bis 3, **dadurch gekennzeichnet, dass** intrazellulärer Sauerstoff (O₂) in den Algen gebunden wird,
der intrazelluläre Sauerstoff (O₂) durch Zugabe zumindest eines Bindemittels gebunden wird,. wobei
das bzw. die Bindemittel nach der Aufnahme von Sauerstoff (O₂) regeneriert wird bzw. werden.

5. Verfahren nach einem der Ansprüche 1 bis 4, **dadurch gekennzeichnet, dass** intrazellulärer Sauerstoff (O₂) biochemisch gebunden wird, wobei
als Bindemittel Myoglobin zugegeben wird und/oder
als Bindemittel Porphorin zugegeben wird.

6. Verfahren nach einem der Ansprüche 1 bis 5, **dadurch gekennzeichnet, dass** der intrazelluläre Sauerstoff (O₂) chemisch gebunden wird, wobei
als Bindemittel Hydrazin und/oder ein Hydrazinsalz, zugegeben wird, und/oder
als Bindemittel ein Terpen zugegeben wird.

7. Verfahren nach einem der Ansprüche 1 bis 6, **dadurch gekennzeichnet, dass *zur*** Abtrennung der gasförmigen Zwischenprodukte Wasserstoff (H₂) und Sauerstoff (O₂) von dem Algenmedium eine
schwarze Membran (3), ***und*/*oder***
eine poröse Membran (3) aus CLPE (cross-linked polyethylene) ***verwendet wird, und*/*****oder** dass*
die Membran (3) beidseitig in eine hexagonal dichteste Kugelpackung festgelegt wird, und/oder ***dass***
eine CLPE-Membran (3) mit einer inneren Schicht aus PATBS (Poly(acrylamid-tert-butyl-sulfonsäure)) verwendet wird, und/oder ***dass***
eine mehrlagige Membran verwendet wird, deren Lagen zumindest bereichsweise miteinander verschweißt sind, und/oder ***dass***
der osmotische Druck von Kohlendioxid (CO₂) auf der Gasseite der Membran (3) erhöht wird.

8. Verfahren nach einem der Ansprüche 1 bis 7, **dadurch gekennzeichnet, dass** überschüssiger Wasserstoff (H₂) und Sauerstoff (O₂) ***unter Nutzung von Abwärme aus dem Hydrogenese-Schritt*** zur Erzeugung reinen Süßwassers (H₂O) verwendet werden.

9. Verfahren nach einem der Ansprüche 1 bis 8 , **dadurch gekennzeichnet, dass** die zur Erzeugung von Methan (CH₄) verwendeten Methanogenese-Bakterien in einer wässrigen Lösung bereitgestellt werden, welcher periodisch oder fortlaufend geeignete Nährstoffe (5') zugeführt werden, und/oder
dass die zur Erzeugung von Methan (CH₄) verwendeten Methanogenese-Bakterien eine oder eine Mischung der Arten Methanobacterium thermoautotropicum, Methanobacillus, Methanobacterium, Methanococcus, Methanosarcina und Methanothrix aufweisen, und/oder
dass Wasserstoff (H₂) und Kohlendioxid (CO₂) den Methanogenese-Bakterien unter anaeroben Bedingungen und/oder bei einer Temperatur von ungefähr 60 Grad Celsius zugeführt werden, und/oder
dass intrazellulärer Sauerstoff (O₂) in den Methanogenese-Bakterien gebunden wird.

10. Verfahren nach einem der Ansprüche 1 bis 9, **dadurch gekennzeichnet, dass** die Abtrennung des Methans (CH₄) von dem Bakterienmedium über eine Membran (3'), insbesondere eine poröse Membran (3') aus CLPE (cross-linked polyethylene) erfolgt.

11. *Verfahren nach Anspruch 10, **dadurch gekennzeichnet, dass***
*die Membran (3') beidseitig in eine hexagonal dichteste Kugelpackung festgelegt wird, und*/*oder dass*
*eine CLPE-Membran* (3) *mit einer inneren Schicht aus PATBS (Poly(acrylamid-tert-butyl-sulfonsäure)) verwendet wird, und*/*oder dass*
*eine mehrlagige Membran verwendet wird, deren Lagen zumindest bereichsweise miteinander verschweißt sind, und*/*oder dass*
*der osmotische Druck von Kohlendioxid (CO₂) auf der Gasseite der Membran (3') erhöht wird.*

12. Verfahren nach einem der Ansprüche 1 bis 11, **dadurch gekennzeichnet, dass** den Algen und/oder den Methanogenese-Bakterien zugeführtes Kohlendioxid (CO₂) aus Trockeneis gewonnen wird, und/oder
dass den Algen und/oder den Methanogenese-Bakterien zugeführtes Kohlendioxid (CO₂) aus kohlendioxidreichen Gasströmen bereitgestellt wird, und/oder
dass nicht verbrauchtes bzw. nicht umgesetztes Kohlendioxid (CO₂) - zurückgewonnen und in den Prozess rückgeführt wird.

13. Verfahren nach einem der Ansprüche 1 bis 12, **dadurch gekennzeichnet, dass** entstehendes überschüssiges Algen- und/oder Bakterienmaterial periodisch oder fortlaufend entnommen wird und einem Biogasprozess zur zusätzlichen Methangewinnung zugeführt wird.

14. Verfahren nach einem der Ansprüche 1 bis 13, **dadurch gekennzeichnet, dass** Wärme aus dem Hydrogenese-Schritt abgeführt wird.

15. *Verfahren nach Anspruch 14, **dadurch gekennzeichnet, dass** zwischen dem Hydrogenese-Schritt und dem Methanogenese-Schritt Wärme übertragen wird.*

## Claims

1. A method for the biological generation of methane (CH₄), the method comprising the steps:
- generation of hydrogen (H₂) and oxygen (O₂) from carbon dioxide (CO₂) and water (H₂O) by green algae under the action of light (hydrogenesis),
- separation of the gaseous intermediates hydrogen (H₂) and oxygen (O₂) from the algal medium via a membrane (3),
- separation of the oxygen (O₂) generated from the hydrogen (H₂) generated,
- generation of methane (CH₄) from the hydrogen (H₂) generated and carbon dioxide (CO₂) by methanogenesis bacteria (methanogenesis),
- separation and, where applicable, liquefaction of the methane (CH₄) generated.

2. A method according to claim 1, **characterised in that** the algae used for the generation of hydrogen (H₂) are separated from the light source, especially sunlight, by a substantially transparent plate (6).

3. A method according to claim 2, **characterised in that** a plate (6) having a self-cleaning effect (lotus effect), provided by a silane coating, on the side remote from the light is used.

4. A method according to any one of claims 1 to 3, **characterised in that** intracellular oxygen (O₂) in the algae is bound,
the intracellular oxygen (O₂) being bound by the addition of at least one binder,
the binder or binders being regenerated after the absorption of oxygen (O₂).

5. A method according to any one of claims 1 to 4, **characterised in that** intracellular oxygen (O₂) is bound biochemically,
with myoglobin being added as binder and/or
with porphorin being added as binder.

6. A method according to any one of claims 1 to 5, **characterised in that** the intracellular oxygen (O₂) is bound chemically,
with hydrazine and/or a hydrazine salt being added as binder, and/or
with a terpene being added as binder.

7. A method according to any one of claims 1 to 6, **characterised in that**, for separation of the gaseous intermediates hydrogen (H₂) and oxygen (O₂) from the algal medium,
a black membrane (3) and/or
a porous membrane (3) made of CLPE (cross-linked polyethylene) is used, and/or the membrane (3) is fixed on both sides into a hexagonally closest sphere packing, and/or
a CLPE membrane (3) having an inner layer made of PATBS (poly(acrylamide tert.-butylsulphonic acid)) is used, and/or
a multi-layer membrane the layers of which are welded to one another at least in regions is used, and/or
the osmotic pressure of carbon dioxide (CO₂) on the gas side of the membrane is increased.

8. A method according to any one of claims 1 to 7, **characterised in that** excess hydrogen (H₂) and oxygen (O₂) are used to generate pure fresh water (H₂O) using the waste heat from the hydrogenesis step.

9. A method according to any one of claims 1 to 8, **characterised in that** the methanogenesis bacteria used for the generation of methane (CH₄) are provided in an aqueous solution to which suitable nutrients (5') are periodically or continuously supplied, and/or
the methanogenesis bacteria used for the generation of methane (CH₄) have one of the species or a mixture of the species Methanobacterium thermoautotropicum, Methanobacillus, Methanobacterium, Methanococcus, Methanosarcina and Methanothrix, and/or
hydrogen (H₂) and carbon dioxide (CO₂) are supplied to the methanogenesis bacteria under anaerobic conditions and/or at a temperature of approximately 60 degrees Celsius, and/or
intracellular oxygen (O₂) in the methanogenesis bacteria is bound.

10. A method according to any one of claims 1 to 9, **characterised in that** the separation of the methane (CH₄) from the bacterial medium is carried out via a membrane (3'), especially a porous membrane (3') made of CLPE (cross-linked polyethylene).

11. A method according to claim 10, **characterised in that**
the membrane (3') is fixed on both sides into a hexagonally closest sphere packing, and/or
a CLPE membrane (3') having an inner layer made of PATBS (poly(acrylamide tert.-butylsulphonic acid)) is used, and/or
a multi-layer membrane the layers of which are welded to one another at least in regions is used, and/or
the osmotic pressure of carbon dioxide (CO₂) on the gas side of the membrane (3') is increased.

12. A method according to any one of claims 1 to 11, **characterised in that** carbon dioxide (CO₂) supplied to the algae and/or to the methanogenesis bacteria is obtained from dry ice, and/or
carbon dioxide (CO₂) supplied to the algae and/or to the methanogenesis bacteria is provided from carbon dioxide-rich gas streams, and/or
unused or unreacted carbon dioxide (CO₂) is recovered and returned to the process

13. A method according to any one of claims 1 to 12, **characterised in that** excess algal and/or bacterial material produced is periodically or continuously removed and fed to a biogas process for obtaining additional methane.

14. A method according to any one of claims 1 to 13, **characterised in that** heat from the hydrogenesis step is conveyed away.

15. A method according to claim 14, **characterised in that** heat is transferred between the hydrogenesis step and the methanogenesis step.

## Revendications

1. Procédé de production biologique de méthane (CH₄), le procédé comprenant les étapes suivantes :
• production d'hydrogène (H₂) et d'oxygène (O₂) à partir de dioxyde de carbone (CO₂) et d'eau (H₂O) par des algues vertes moyennant l'action de la lumière (hydrogenèse),
• séparation des produits intermédiaires gazeux que sont l'hydrogène (H₂) et l'oxygène (O₂) du milieu des algues, à travers une membrane (3).
• séparation de l'oxygène produit (O₂) d'avec l'hydrogène produit (H₂),
• production de méthane (CH₄) à partir de l'hydrogène produit (H₂) et du dioxyde de carbone (CO₂) par des bactéries capables de méthanogenèse (méthanogenèse),
• séparation et, le cas échéant, liquéfaction du méthane produit (CH₄).

2. Procédé selon la revendication 1, **caractérisé en ce que** les algues utilisées pour la production de l'hydrogène (H₂) sont séparées de la source de lumière, en particulier de la lumière du soleil, par une vitre (6) sensiblement transparente.

3. Procédé selon la revendication 2, **caractérisé en ce qu'**on utilise une vitre (6) qui présente du côté détourné de la lumière un effet autonettoyant (effet lotus) qui est fourni par un revêtement de silane.

4. Procédé selon l'une quelconque des revendications 1 à 3, **caractérisé en ce que** de l'oxygène (O₂) intracellulaire est lié dans les algues, sachant que
l'oxygène intracellulaire (O₂) est lié par addition d'au moins un agent de liaison, sachant que
le ou les agents de liaison est ou sont régénéré(s) après l'absorption de l'oxygène (O₂).

5. Procédé selon l'une quelconque des revendications 1 à 4, **caractérisé en ce que** l'oxygène (O₂) intracellulaire est lié biochimiquement, sachant que
l'on ajoute comme agent de liaison de la myoglobine et/ou que l'on ajoute comme agent de liaison de la porphorine.

6. Procédé selon l'une quelconque des revendications 1 à 5, **caractérisé en ce que** l'oxygène (O₂) intracellulaire est lié chimiquement, sachant que
l'on ajoute comme agent de liaison de l'hydrazine et/ou un sel de l'hydrazine, et/ou
que l'on ajoute comme agent de liaison un terpène.

7. Procédé selon l'une quelconque des revendications 1 à 6, **caractérisé en ce que**, pour la séparation des produit gazeux intermédiaires que sont l'hydrogène (H₂) et l'oxygène (O₂) d'avec le milieu des algues, une
membrane (3) noire et/ou
une membrane (3) poreuse en CLPE (cross-linked polyethylene) = polyéthylène réticulé) sont utilisées, et/ou **en ce que**
la membrane (3) est immobilisée des deux côtés dans un empilement hexagonal de sphères de densité maximale et/ou **en ce que**
une membrane (3) en CLPE avec une couche intérieure en PAT13S (acide poly(acrylamido-tertiobutyl-sulfonique)) est utilisée, et/ou **en ce que**
une membrane en plusieurs couches est utilisée, ses couches étant soudées ensemble au moins par endroit, et/ou **en ce que**
la pression osmotique du dioxyde de carbone (CO₂) du côte gaz de la membrane (3) est augmentée.

8. Procédé selon l'une quelconque des revendications 1 à 7, **caractérisé en ce que** l'hydrogène (H₂) en excédent et l'oxygène (O₂) sont utilisés à la production d'eau douce pure (H₂O) moyennant l'exploitation de la chaleur résiduelle provenant de l'étape d'hydrogenèse.

9. Procédé selon l'une quelconque des revendications 1 à 8, **caractérisé en ce que** les bactéries de méthanogenèse utilisées pour la production de méthane (CH₄) sont fournies dans une solution aqueuse, à laquelle des nutriments (5) appropriés sont acheminés périodiquement ou continûment, et/ou
**en ce que** les bactéries de méthanogenèse utilisées pour la production de méthane (CH₄) comportent une des espèces ou un mélange des espèces Methanobacterium thermoautotropicum, Methanobacillus, Metanococcus, Methanosarcina et Methanothrix, et/ou
**en ce que** l'hydrogène (H₂) et le dioxyde de carbone (CO₂) sont acheminés aux bactéries de méthanogenèse sous des conditions anaérobies et/ou à une température d'environ 60 degrés Celsius, et/ou
**en ce que** de l'oxygène (O₂) intracellulaire est lié dans les bactéries de méthanogenése.

10. Procédé selon l'une quelconque des revendications 1 à 9, **caractérisé en ce que** la séparation du méthane (CH₄) d'avec le milieu des bactéries s'effectue à travers une membrane (3'), en particulier une membrane (3') poreuse en CLPE (cross-linked polyethylene).

11. Procédé selon la revendication 10, **caractérisé en ce que**
la membrane (3') est immobilisée des deux côtés dans un empilement hexagonal de sphères de densité maximale et/ou **en ce que**
une membrane (3') en CLPE avec une couche intérieure en PATBS (acide poly(acrylamido-tertiobutyl-sulfonique)) est utilisée, et/ou **en ce que**
une membrane en plusieurs couches est utilisée, ces couches étant soudées ensemble au moins par endroit, et/ou **en ce que**
la pression osmotique du dioxyde de carbone (CO₂) du côte gaz de la membrane (3) est augmentée.

12. Procédé selon l'une quelconque des revendications 1 à 11, **caractérisé en ce que** le dioxyde de carbone (CO₂) acheminé aux algues et/au aux bactéries de méthanogenèse est obtenu à partir de carboglace, et/ou
**en ce que** le dioxyde de carbone (CO₂) acheminé aux algues et/ou aux bactéries de méthanogenèse est fourni à partir de courants de gaz riches en dioxyde de carbone, et/ou
**en ce que** le dioxyde de carbone (CO₂) non consommé, respectivement n'ayant pas réagi, est récupéré et est recyclé dans le processus.

13. Procédé selon l'une quelconque des revendications 1 à 12, **caractérisé en ce que** le matériau d'algues ou de bactéries est prélevé périodiquement ou continument et est acheminé à un processus de biogaz pour l'obtention de méthane supplémentaire.

14. Procédé selon l'une quelconque des revendications 1 à 13, **caractérisé en ce que** la chaleur provenant de l'étape de méthanogenèse est évacuée.

15. Procédé selon la revendication 14, **caractérisé en ce que** de la chaleur est transférée entre l'étape d'hydrogenèse et l'étape de méthanogenèse.
